# EUROPEAN PATENT APPLICATION

(11) **EP 2 113 200 A1**
(43) Date of publication of application: **04.11.2009**
(21) Application number: 09159136.2
(22) Date of filing: 30.04.2009
(51) Int. Cl.: A61B 6/14

(54) **A system and method for automatic jaw measurement for panoramic radiology**

(30) Priority: 30.04.2008 US 112166
(71) Applicant: Schick Technologies, Inc., Long Island City NY 11101 (US)
(72) Inventor: Mandelkern, Stan, Teaneck, NJ 07666 (US); Michaeli, Daniel, Riverdale, NY 10471 (US)
(74) Representative: Sommer, Peter

(57) **Abstract**

A method of conducting a panoramic x-ray examination. The method includes generating a signal representing an anatomical region of interest, for instance, via a pressure map, calculating an imaging layer based on the generated signal, and generating a scan based on the calculated imaging layer.

## Description

### BACKGROUND OF THE INVENTION

### Field Of The Invention

The present invention generally relates to the field of digital radiology systems, and more particularly to dental radiology systems which provide panoramic radiology.

### Related Art

Panoramic x-ray systems are widely employed in the dental industry. Such systems can be useful for detecting potentially harmful conditions in a patient such as, for example, carious lesions, periodontal bone loss, large deposits of calculus, impacted teeth, and jaw fractures. Additionally, such systems can, for example, provide critical information for use in assessing implant viability. Furthermore, such systems can be used to assess growth and development, as is critical in orthodontia. Such systems can also provide an excellent mechanism to aid in a practitioner's general clinical assessment of a patient's mouth.

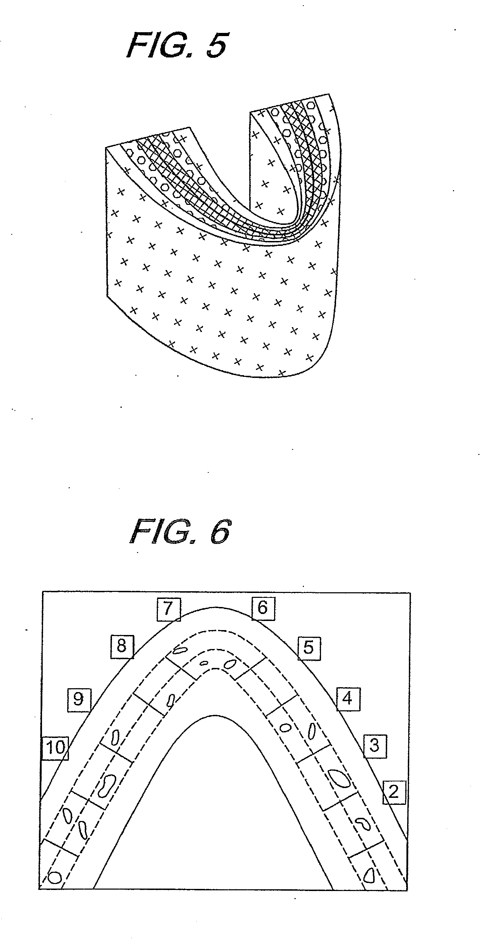

A panoramic x-ray machine typically creates an image by using motion to blur anatomy in front of and behind the area of interest. In a typical panoramic examination, the patient remains stationary while the panoramic x-ray source and the image receptor move in opposite directions from each other around an elliptical path, generating an image through tomography. As the machine rotates, the rotation center is varied, generating an elliptical in-focus region approximating the basic shape of the patient's dental arches. The in-focus region is typically determined by adjusting the velocity of rotation and the magnification factor. This assumed volume in space is generally referred to as the "focal trough" or "imaging layer." Fig. 5 illustrates the general shape of a focal trough used in panoramic machines.

The in-focus region, of course, should correspond to the clinical anatomy of interest. However, it has been discovered that positioning the dentition properly within the image layer can present one of the greatest clinical challenges in panoramic radiography and can be critical to image quality. Accordingly, there is a need to provide a system and method which can provide accurate and precise positioning of the dentition.

### SUMMARY OF THE INVENTION

The present invention meets the above-identified needs by providing, in at least one embodiment, a system, method, and apparatus that achieves automatic jaw measurement for panoramic radiology.

Before describing the present invention in detail, it is to be understood that the practice of the present invention employs, unless otherwise indicated, conventional methods of panoramic x-ray imaging and processing as known by those having ordinary skill in the art. The present invention is not limited to particular formulations or process parameters as such may, of course, vary.

The present invention in accordance with one embodiment provides a method of conducting a panoramic x-ray examination. The method includes generating a signal representing a pressure map of an anatomical region of interest, calculating an imaging layer based on the generated signal, and generating a scan based on the calculated imaging layer.

The anatomical region of interest may represent at least one of a dental arch and a location of a maxilla and a mandible. The first generating step may be performed using a pressure transducer. The method may further include performing the scan using a motion platform. The motion platform may include an x-ray source and an x-ray detector, and the x-ray detector may be a solid-state sensor.

The method may further include transmitting a signal obtained by the scan to an image processing system, and may further include generating tooth location information using the generated signal.

The present invention in accordance with another embodiment provides a panoramic x-ray system, including (1) a pressure transducer adapted to generate a signal representing a pressure map of an anatomical region of interest, (2) a processor adapted to calculate an imaging layer based on the generated signal, and (3) a motion platform adapted to perform a scan using the calculated imaging layer. The processor programs the motion platform based on the calculated imaging layer.

The present invention in accordance with another embodiment provides a method of conducting a panoramic x-ray examination. The method includes generating a signal representing a pressure map of an anatomical region of interest, and matching an imaging layer to the anatomical region of interest using the generated signal.

The present invention in accordance with another embodiment provides a method of conducting a panoramic x-ray examination. The method includes generating an optical impression image of an anatomical region of interest, calculating an imaging layer based on the optical impression image, and generating a scan based on the calculated imaging layer.

The first generating step may be performed using an intra-oral camera. The method may further include performing the scan using a motion platform.

The present invention in accordance with another embodiment provides a method of conducting a panoramic x-ray examination, comprising the steps of generating a signal representing an anatomical region of interest, and matching an imaging layer to the anatomical region of interest using the generated signal.

The generating step may be performed using an x-ray image. The generating step may include using a plurality of position-sensitive sensors creating the signal representing the anatomical region of interest. The generating step may include using a pressure transducer creating the signal representing the anatomical region of interest.

Further features and advantages of the present invention as well as the structure and operation of various embodiments of the present invention are described in detail below with reference to the accompanying drawings.

### BRIEF DESCRIPTION OF THE DRAWINGS

The features and advantages of the present invention will become more apparent from the detailed description set forth below when taken in conjunction with the drawings in which like reference numbers indicate identical or functionally similar elements.

Figs. 1a and 1b are drawings showing respective panoramic imaging apparatuses having known positioning elements.

Fig. 2 is a system-level schematic of a panoramic radiology system according to an embodiment of the present invention.

Fig. 3 is a flowchart showing a method for automatic jaw measurement for panoramic radiology according to one embodiment of the present invention.

Fig. 4 is an architecture diagram of an example of a data processing system or device which can be used in connection with an embodiment of the present invention.

Fig. 5 illustrates the general shape of a focal trough used in panoramic machines.

Fig. 6 illustrates an example of a pressure map used in accordance with one embodiment of the present invention.

### DETAILED DESCRIPTION

Typical positioning aids, as known in the art, are generally placed against a patient's forehead, below a patient's chin, on the lateral aspects of the head (sometimes within the ears), and between the patient's teeth.

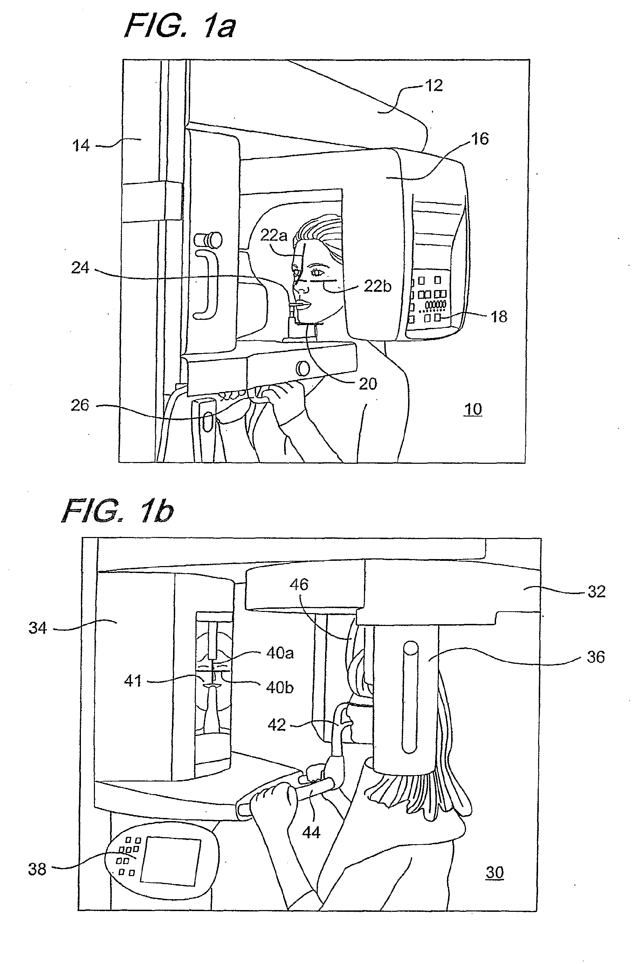

Fig. 1a shows a panoramic x-ray imaging apparatus 10, having known positioning elements for indirectly aligning patient anatomy within a focal trough. In Fig. 1a, a vertical carriage 12 extends from a vertical support 14. A rotary 16 is rotatably attached to the vertical carriage 12, and is provided with a rotary mechanism for rotating itself against the vertical carriage 12 by, for example, a step motor or the like. An x-ray source (not shown in Fig. 1a), provided with an x-ray tube, is disposed at one end of the rotary 16, and an x-ray receptor (not shown in Fig. 1a) is disposed at the other end. The practitioner uses the apparatus control panel 18 in operating the panoramic x-ray apparatus 10, the control panel 18 including a number of switches for allowing the practitioner to perform various functions such as turning an electric current source on and off, controlling movement of the rotary 16, etc.

In Fig. 1a, the patient rests her head on chin support 20, and laser lights 22a, 22b projected from a laser light source (not shown) identify anatomic planes for better positioning. A mirror (see Fig. 1b) can assist the patient in keeping her head aligned with laser lights 22a, 22b. A bite tab 24 aids in positioning as well, since the patient's movement is restricted while biting down on the bite tab 24. Further, the control panel 18 may include buttons and switches for programming body size (e.g., male, female, child, etc.), which information aids in determining the proper placement of the various positioning elements. The patient may also hold on to one or more handles 26, to assist in keeping the patient stationary throughout the examination.

Fig. 1b shows a panoramic x-ray imaging apparatus 30 according to another example, also having known positioning elements for indirectly aligning patient anatomy within the focal trough. In Fig. 1b, a vertical carriage 32 extends from a vertical support 34. A rotary 36 is rotatably attached to the vertical carriage 32, and is provided with a rotary mechanism for rotating itself against the vertical carriage 32 by, for example, a step motor or the like. An x-ray source (not shown in Fig. 1b), provided with an x-ray tube, is disposed at one end of the rotary 36, and an x-ray receptor (not shown in Fig. 1b) is disposed at the other end. The practitioner uses the apparatus control panel 38 in operating the panoramic x-ray apparatus 30, the control panel 38 including a number of switches for allowing the practitioner to perform various functions such as turning on and off an electric current source, controlling movement of the rotary 36, etc.

In Fig. 1b, laser lights 40a, 40b projected from a laser light source (not shown) identify anatomic planes for better positioning, and the mirror 41 can assist the patient in keeping her head aligned with the laser lights 40a, 40b. A bite tab 42 aids in positioning as well, since the patient's movement is restricted while biting down on the bite tab 42. Further, the control panel 38 may include buttons for programming body size (e.g., male, female, child, etc.), which can aid in determining the proper placement of the various positioning elements. The patient may also hold on to the handle 44, which assists in keeping the patient stationary throughout the examination. The patient may also lean her head against head rest 46, which may also aid in the positioning.

In known techniques, based upon such positioning, the machine attempts to inference the location of the patient's dental arch using anatomic norms. However, it has been discovered that patient positioning using techniques such as those shown in Figs. 1a and 1b can be difficult, can be inefficient, and, if the end result is not accurate, can require multiple exposures and unintended dosage.

In particular, Figs. 1a and 1b show examples of known systems that *indirectly* orient a patient within the focal trough. As described above, alignment of anatomic planes is generally performed using external facial landmarks such as the chin, the forehead, and lateral aspects of the head. Anatomic planes can be further adjusted with the use of positioning lights. However, the aforementioned systems shown in Figs. 1a and 1b are based only upon soft tissue landmarks, and can only serve to approximate the location of the upper and lower teeth, the mandible, and the maxilla. The relationship between the soft tissue structures and the desired dental structures varies quite significantly within the population. Manufacturers typically design the mechanics with a tolerance such that a wide imaging layer will encompass the majority of anatomic variation. However, it has been discovered that the wider the image layer is, the greater the image blur may be, i.e., the less clarity the resulting image may have.

Moreover, it is noted that further indirect anatomic information can be obtained in a number of ways. For example, an estimate of the patient's jaw size can be calculated based upon a selected parameter, which may vary depending, for example, on the age of the patient under examination.

In U.S. Patent Application Publication No. 2006/0056582 A1 (*Stoeckl*), a more recent advance is discussed, including a system envisioned to calculate the angle of the occlusal plane. That system includes a pivoting member on which the patient bites. The resulting pivot angle is calculated and utilized to generate the desired inclination of the occlusal plane with respect to the panoramic x-ray source. Based upon the predicted location, size, and shape of the patient's jaw, machine algorithms estimate x-ray tube and receptor travel geometry. However, it has been discovered that by using only soft tissue landmarks and anatomic norms, the aforementioned procedures can offer only an *approximate* location of the dental arch within a panoramic system imaging layer.

On the other hand, it has been discovered that, typically, the sharpest and most clinically accurate panoramic images are generated only when a patient is positioned with the dental arches and condyles coincident with the machine's image layer. Because the actual size and shape of the jaws can be highly variable from patient to patient, it can ultimately be difficult to perform this procedure accurately for each patient. Accordingly, to accommodate potential position inaccuracy, manufacturers typically design with a reasonably wide and forgiving image layer in order to make patient positioning easier and ensure that a range of patient jaw sizes and shapes can be accommodated. Unfortunately, it has been discovered that as the image layer is widened, image clarity can suffer; consequently, a system that could closely match a narrow image layer to the anatomic region of interest would offer a distinct advantage.

In U.S. Patent Application Publication No. 2006/0203959 (*Spartiotis*) a high-frame rate detector for use in a dental panoramic x-ray system is discussed. In that system, multiple frames corresponding to numerous potential image layers are generated. Consequently, the clinician can select from a multitude of potential image layers, and would presumably select the image layer resulting in the greatest clarity and preferred overall image quality. However, it has been discovered that the CdZnTe-CMOS detector discussed in U.S. Patent Application Publication No. 2006/0203959 is unnecessarily complex and extremely expensive, and the use of that detector would not be compatible with film-based machines; furthermore, it would be preferable if the optimal image layer were presented without user intervention in order to improve upon the accuracy.

In U.S. Patent No. 6,424,694 (*Molteni*), a panoramic x-ray system that works along with a tray holding an impression material is discussed. The patient bites into the impression material and a standard impression is made according to procedures well known in the art. The impression material is aligned within the panoramic x-ray system, allowing the dentition, rather than external landmarks, to be utilized. Once alignment is complete, the patient re-bites into the impression material in a final effort to ensure that the patient's anatomy is appropriately positioned. However, while this system may afford certain advantages, it has been discovered that the step of forming an impression is cumbersome, and nevertheless the system still requires an alignment procedure, albeit with an impression rather than with the clinical patient.

The present invention offers a fresh approach, and provides, at least in one embodiment, a system, method, and apparatus for automatic jaw measurement for panoramic radiology.

The present invention in one embodiment provides a system, method, and apparatus for *directly* coinciding the dental structures of a patient with a panoramic x-ray machine's imaging layer. Whereas a variety of *indirect* means are known and employed within commercial systems, it has been discovered that such means are apt to be inaccurate or cumbersome, or both, and result in an image of only modest quality.

The present invention according to one embodiment provides a panoramic radiology system that can directly and precisely coincide the imaging layer with the anatomy to be clinically imaged, by using the actual location of a patient's teeth, and adjusting machine mechanics and imaging geometry for a preferred view of the dental structures. Accordingly, a panoramic radiology system with reduced image blur and distortion can be achieved, which can allow for a low-blur panoramic view of the clinical anatomy of interest.

According to an example embodiment of the present invention, a compliant pressure transducer is used to automatically identify the actual location of a patient's teeth and, therefore, the patient's dental arch. The patient bites down on the pressure transducer, and the pressure transducer generates electrical signals representing a pressure map of the patient's teeth or, in an edentulous patient, the location of the maxilla and mandible. With the identification of the dental arch, the machine's imaging layer can be matched to the particular patient's anatomy by optimizing the x-ray source and detector motion, which can thereby produce an enhanced panoramic image. Accordingly, the present invention in accordance with an example embodiment can provide a panoramic radiology system with simple positioning and accurate imaging. In this example embodiment of the present invention, direct identification of the dental structures can be made, which can enable a higher quality, low blur image.

A pressure transducer is a transducer that converts pressure (for example a fluid pressure) into an electric or mechanical signal. One common type of pressure transducer suitable for use with the present invention is a high spatial resolution matrix of pressure transducers. The conversion of pressure into an electrical signal is achieved by the physical deformation of the transducers which are bonded onto a thin flexible substrate. Pressure applied to the substrate, such as by the patient biting down, produces a measurable change in the electrical properties of the individual pressure sensing elements in the matrix proportional to the pressure at each point. An electrical output from the pressure transducer can be, for example, an analog electric signal, a millivolt or voltage output, a milli-amp output, etc.

One transducer suitable for use with the present invention is manufactured by Tekscan and sold under the name Tekscan T-Scan® III. The transducer can be encapsulated within a sheath, can be disposable, or can otherwise be designed with a biocompatible material. A pressure map can be generated which displays graphically (e.g., in a two-dimensional image, a three-dimensional image, a force vs. time graph, etc.) tooth contact data highlighting each tooth and the force level exerted on that tooth during occlusion. Fig. 6 illustrates an example of a pressure map, generated by an image processing system, which shows a two-dimensional graphical representation of the occlusion. According to the present invention, the data from the pressure map can be used to calculate the image layer required.

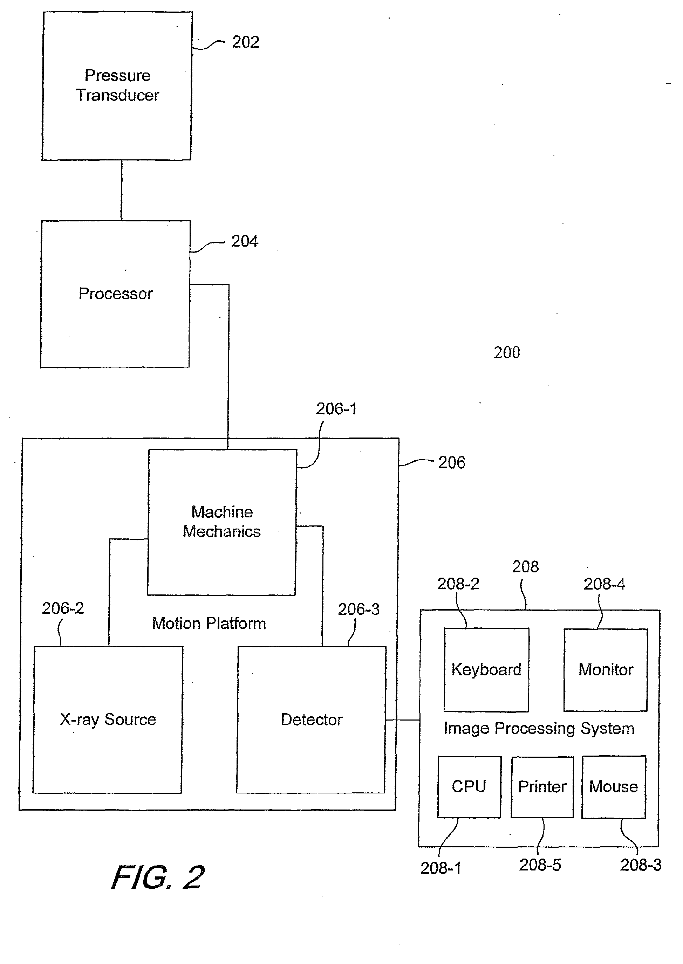

Fig. 2 is a block diagram showing a system 200 for automatic jaw measurement for panoramic radiology according to one embodiment of the present invention. In particular, the system shown in Fig. 2 is a panoramic x-ray system which can enable direct positioning of the patient's dental structures with the panoramic machine's imaging layer. By virtue of the features of the system shown in Fig. 2, the imaging layer can be precisely custom-generated based upon the actual location of the dental structures. This stands in contrast to known systems, in which precise placement is required to match a predetermined imaging layer. It is noted with respect to the system of Fig. 2 that the dental arch location can be utilized in conjunction with those aids orienting the anatomic planes, resulting in higher-quality results. That is, the physical features of a system according to one embodiment of the present invention, including for example the pressure transducer 202, may incorporate and work in conjunction with some or all of the features shown in Figs. 1a and 1b.

In particular, in Fig. 2, a hygienic pressure transducer 202 is used to automatically identify the actual location of a patient's teeth and, therefore, the patient's dental arch. A patient bites down on the pressure transducer 202, and the pressure transducer 202 generates electrical signals representing a pressure map of the patient's teeth or, in an edentulous patient, the location of the maxilla and mandible. According to the present invention, the purpose of the pressure transducer 202 is to identify the actual, not approximate, location of the anatomic region of interest.

Once the pressure map is generated by the pressure transducer 202, and the location of the dental arch is known, the processor 204 (which may include an analog-to-digital converter and other suitable circuit components) calculates the image layer required, and programs the motion platform 206 of the panoramic radiology system 200 to direct the motion platform 206 pursuant to the location of the dental arch. The image layer can be calculated in a variety of ways. In one embodiment, for example, a mean best-fit pathway of occlusal surfaces (opposing teeth) can be computed using a spline function. Data from the pressure map can be plotted on x-y coordinates, and a best-fit curve can be determined from an algorithm, for example. Of course, as a person having ordinary skill in the art would readily appreciate, the present invention is not limited to this example, and a computational best-fit shape function can be generated in a variety of ways.

The processor 204 sends programming signals to the machine mechanics unit 206-1 of the motion platform 206, which directs the x-ray source 206-2 and the x-ray detector 206-3 accordingly. The elliptical focal trough can be recreated using, for example, a series of stepper motors directing the x-ray source 206-2 and the x-ray detector 206-3. Of course, it is still preferable to position or re-position the patient's head accurately so that the machine mechanics unit 206-1 can operate in a more ideal fashion to recreate the pathway.

The x-ray detector or receiver 206-3 may be x-ray film, a stored phosphor device, a digital sensor or receptor such as a CMOS or time-delay integration (TDI) CCD, etc. Accordingly, the electronic sensor may include a charge-coupled device (CCD), a CMOS active pixel sensor (APS) array, or another type of radiation sensor.

Solid-state sensors which convert x-rays into an electrical signal are increasingly being used in place of photographic film, and such radiography systems offer many advantages over traditional film-based radiography. For example, the image of the anatomy may be generated by a computer almost instantaneously, thus improving work-flow and eliminating the entire film development process, including the use of potentially harmful chemicals. In addition, because the images are generated electronically, they can be stored in and accessed from a computer database. Digital sensors may also be wired or wireless.

In any event, a scan of the patient is performed and the digital data can be transmitted to an image processing system 208, from which it can be presented to the clinician and to the patient. Both standard as well as specialized panoramic imaging procedures such as a TMJ (Temporomandibular) study or a TSA (Transversal Sections) slice analysis can benefit from this technique.

The image processing system 208 can include a central processing unit (CPU) 208-1 and can process the signal to produce an image on an associated output device (such as the monitor 208-4 or the printer 208-5). The image processing system 208 allows the user to view and analyze the dental images that the system creates. The image processing system 208 may be, for example, a desktop, tower, laptop, or notebook computer, equipped with software for processing the data provided to it by the sensor 206-3. The image processing system 208 may be connected to or have built in one or more input devices, such as a keyboard 208-2 and a mouse 208-3, and one or more output devices, such as the display or monitor 208-4 and the printer 208-5.

These devices allow the user to view and analyze the dental images that the system creates through a graphical user interface, and can also allow the user to control the operation of the system. For example, an interface screen can enable a user to easily access the information and initiate analysis. The image processing system can also include or be connected to a storage device (not shown), such as a hard drive, for permanent storage of the images in patient files. Other potential storage devices include floppy disks, ZIP drives, magnetic tape, and optical medium. A variety of computer program products comprising, in general, a computer-readable medium, can be used with the present invention.

The software might run on a PC-compatible, Macintosh®, or Unix®-based computer, among others. In one embodiment the software runs on a PC-compatible computer with a Pentium®-based CPU running Windows 98®, Me®, 2000®, XP®, or Vista®. Of course, these examples are not meant to be limiting in any way, and the software can be written to be compatible with other or newer operating systems as well. In another embodiment, the software can be written to be complementary to that used for acquiring intra-oral images and for standard panoramic, video, and cephalometric examinations. The software also can preferably be compatible with dental practice management software.

The computer preferably contains at least 64 MP of RAM and, for example, 20 GB of hard disk space to store the software and image files. The display would preferably be optimized for video images at color. It might also be advantageous to bundle the system with a photo-quality printer for printing examinations and a backup system for storing image and patient data.

It is noted that the present invention is of course not limited to the details shown in Fig. 1. For example, while in the example embodiment shown in Fig. 1, the processor 204 is shown as a separate unit from the image processing system 208, it is of course to be understood that the functions performed by the processor 204 can also be performed by the image processing system 208; accordingly, the processor 204 and the image processing system 208 can be combined into a single unit.

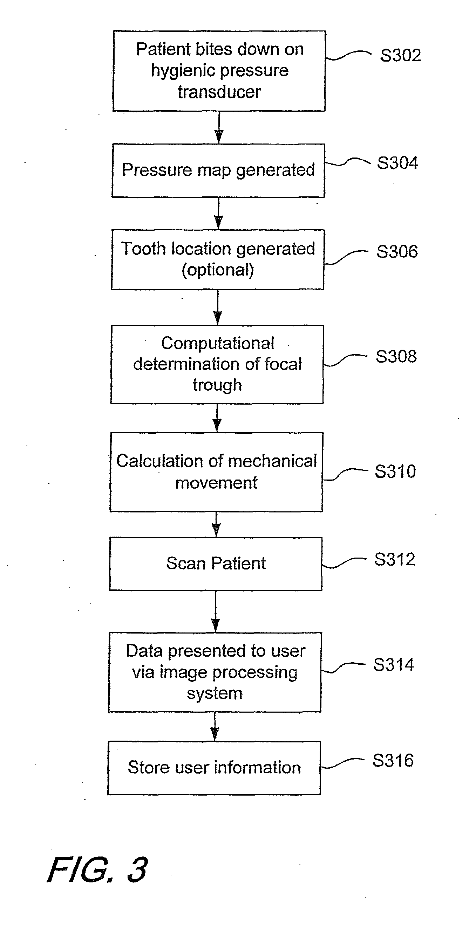

Fig. 3 is a flowchart showing a method 300 for automatic jaw measurement for panoramic radiology according to one embodiment of the present invention. The method 300 can use a system such as system 200 of Fig. 2. By virtue of the method shown in Fig. 3, the imaging layer can be precisely custom-generated based upon the actual location of the dental structures. The dental arch location can also be utilized in conjunction with those aids orienting the anatomic planes, resulting in higher quality result.

In Fig. 3, an examination proceeds as shown, and advantageously employs the system described above, although it is not limited to that system. In step S302, a patient bites down on a hygienic pressure transducer, such as the pressure transducer 202, which is used to automatically identify the actual location of a patient's teeth and, therefore, the patient's dental arch. In step S304, the patient bites down on the pressure transducer 202, and the pressure transducer 202 generates a pressure map of the patient's teeth or, in an edentulous patient, the location of the maxilla and mandible. The purpose of the pressure transducer 202 is to identify the actual, not approximate, location of the anatomic region of interest.

In step S306, the resulting impression can also be used to generate tooth location information, to be subsequently tagged to the digital x-ray image, and useful in examining and diagnosing the patient. This step is optional.

Once the pressure map is generated in step S304 by the pressure transducer 202, and therefore the location of the dental arch is known, in step S308 a computational determination of the focal trough is carried out, for example by a processor such as the processor 204 of Fig. 2. In step S310, the mechanical movement of the gantry of the panoramic radiology system is calculated, and a motion platform (such as the motion platform 206 of Fig. 2, including the x-ray source 206-2 and the x-ray detector 206-3) is programmed. Accordingly, the motion platform 206 is directed pursuant to the location of the dental arch, and the elliptical focal trough can be recreated.

In step S312, a scan of the patient is performed and the digital data can be transmitted to an image processing system (such as the image processing system 208 of Fig. 2) from which it can be presented to the clinician (step S314) and stored (s316). The method as illustrated in Fig. 3 then ends.

It is of course to be understood that the present invention is not limited to the examples and embodiments presented. For example, it is noted with respect to the pressure map generated in step S304 that this information may also have other uses in diagnosing and treating patients. For example, the information may be useful in implant selection and planning. Panoramic x-ray imaging systems are often useful for the selection and planning of a surgical implant procedure. Accordingly, information regarding an occlusal surface may be useful for such planning.

Furthermore, it is also noted, with respect to the pressure map generated in step S304, that the present invention is not limited to identifying the location of the dental arch in this manner, and there may be other techniques of identifying the location of the dental arch.

For example, as will be readily understood by a person having ordinary skill in the art, according to another embodiment of the present invention, a camera or other hand-held digitizer can be used to form an optical impression of a patient's dental arches, and the motion platform 206 can be programmed based on the image layer that the processor 204 calculates from the digital image. Optical impression images are typically calculated with an intra-oral camera, which can be used to identify the occlusal surfaces of the teeth, often for the purpose of generating a tooth restoration. One such device or camera suitable for use with the present invention is sold under the name Cerec®, by Sirona Dental Systems. In this embodiment, the clinician can trace the pathway of the dental arch with the camera. The clinician moves the camera over the teeth, creating a digital or optical impression of the location of the teeth in relation to a spatial reference point. Points on the digital or optical impression can define where the teeth are, from which data a best-fit pathway can be generated by the processor 204. The motion platform 206 can be programmed based on the determined best-fit pathway.

According to another embodiment of the present invention, a primary scout x-ray examination may be utilized, in which the actual location of teeth in x-ray space is utilized for the subsequent clinical examination. In particular, a primary scout examination can determine where to scan an image. A digital image is then created, having data showing the location of the teeth, from which data a best-fit pathway is generated by the processor 204. Accordingly, the primary scout x-ray examination can generate an x-ray image representing the location of a patient's teeth, and this image can be used by the processor 204 to calculate an image layer for programming the motion platform 206.

According to yet another embodiment of the present invention, position-sensitive sensors may be placed inside the patient's mouth to locate various positions along the dental arch. Such position-sensitive sensors may be, for example, ferromagnetic sensors. Ferromagnetic sensors placed in the patient's mouth can create a magnetic field, and the location of the teeth can be determined from the changes in the magnetic field sensed when the patient bites down on the ferromagnetic material. A digital map of the location of the teeth can be created, and the motion platform 206 can be programmed based on the image layer that the processor 204 calculates from the digital map.

Of course, the foregoing are examples only, and it is of course to be understood that the present invention is not limited to only the examples presented. In any event, the overall goal is to identify the actual, not approximate, location of the anatomic region of interest.

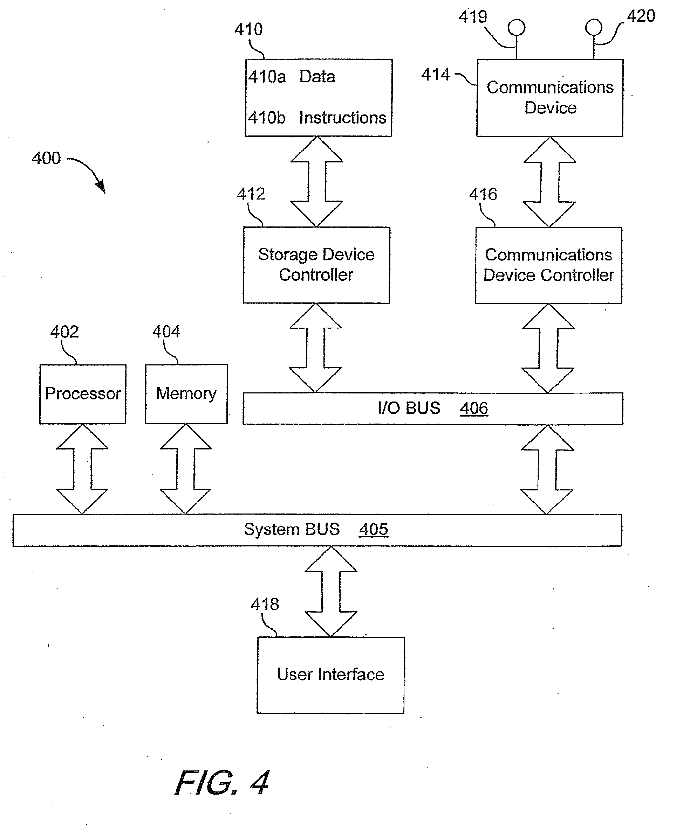

Fig. 4 is an architecture diagram of an example data processing system or device 400, which, according to an example embodiment, can form individual ones of the components 204, 206-1, and 208 of Fig. 2. Data processing system 400 includes a processor 402 coupled to a memory 404 via system bus 406. Processor 402 is also coupled to external Input/Output (I/O) devices (not shown) via the system bus 406 and an I/O bus 408, and at least one input/output user interface 418. Processor 402 may be further coupled to a communications device (interface) 414 via a communications device controller 416 coupled to the I/O bus 408. Processor 402 uses the communications device 414 to communicate with a network, such as, for example, a network as shown in Fig. 2, and the device 414 may have one or more input and output ports. Device 414 has a data port 419 operably coupled to a network for sending and receiving data, and may also have one or more additional input and output ports. A storage device 410 having a computer-readable medium is coupled to the processor 402 via a storage device controller 412 and the I/O bus 408 and the system bus 406. Processor 402 also can include an internal clock (not shown) to keep track of time, periodic time intervals, and the like.

The input/output user interface 418 may include, for example, at least one of a keyboard, a mouse, a trackball, touch screen, a keypad, and/or any other suitable type of user-operable input device(s), and at least one of a video display, a liquid crystal or other flat panel display, a speaker, a printer, and/or any other suitable type of output device for enabling a user to perceive outputted information.

Processing can be performed, for example, by a processor that communicates with a pressure transducer (or intra-oral camera, etc.) and the panoramic x-ray machine, by a processor embedded in the machine, or by any other suitable arrangement. The processor can read the data from, e.g., the pressure transducer and generate a motion profile for the panoramic x-ray machine. Modulation of kV of the x-ray source may also be implemented.

Storage device 410 having a computer readable medium is coupled to the processor 402 via a storage device controller 412 and the I/O bus 408 and the system bus 406. The storage device 410 is used by the processor 402 and controller 412 to store and read/write data 410a, and to store program instructions 410b used to implement at least part of the procedures described herein and shown in Fig. 3. The storage device 410 also stores various routines and operating programs (e.g., Microsoft Windows, UNIX®/LINUX®, or OS/2®) that are used by the processor 402 for controlling the overall operation of the system 400. At least one of the programs (e.g., Microsoft Winsock®) stored in storage device 410 can adhere to TCP/IP protocols (i.e., includes a TCP/IP stack), for implementing a known method for connecting to the Internet or another network, and may also include web browser software, such as, for example, Microsoft Internet Explorer (IE) and/or Netscape Navigator, for enabling a user of the system 400 to navigate or otherwise exchange information with the World Wide Web (WWW).

In operation, processor 402 loads the program instructions 410b from the storage device 410 into the memory 404. Processor 402 then executes the loaded program instructions 410b to perform any of the example methods described herein, for operating the system 200.

The present invention or any part(s) or function(s) thereof may be implemented using hardware, software or a combination thereof and may be implemented in one or more computer systems or other processing systems. It is noted that the various components of the present invention may be controlled by one or more modules coupled to the various components. The modules can operate in accordance with software control programs and operating routines stored in an associated memory or memories. The modules and their sub-modules can write and/or read information to/from the memory or memories, and in this way, can perform operations in accordance with the system, method, and apparatus of the present invention. The modules may be implemented using hardcoded computational modules or other types of circuitry, or a combination of software and circuitry modules. Software routines for performing the modules can, in one embodiment, be stored as instructions in a memory and can be executed by a processor of a control module.

In an embodiment where the invention is implemented using software, the software may be stored in a computer program product, a computer program medium, or a computer-readable medium, and loaded into a computer system using a removable storage drive, a hard drive, or a communications interface. The control logic (software), when executed by a processor, causes the processor to perform the functions of the invention as described herein.

In this document, the terms "computer program medium" and "computer usable medium" are used to refer generally to media such as a removable storage drive, a hard disk installed in a hard disk drive, and signals. Also, "computer-readable medium" is used to refer generally to media such as a storage drive, CD, hard drive or other tangible object that can store a program. These computer program products provide software to the system.

While various embodiments of the present invention have been described above, it should be understood that they have been presented by way of example, and not limitation. It will be apparent to persons skilled in the relevant art(s) that various changes in form and detail can be made therein without departing from the spirit and scope of the present invention. Thus, the present invention should not be limited by any of the above described exemplary embodiments, but should be defined only in accordance with the following claims and their equivalents.

## Claims

1. A method of conducting a panoramic x-ray examination, comprising the steps of:
generating a signal representing a pressure map of an anatomical region of interest (S304);
calculating an imaging layer based on the generated signal (S308); and
generating a scan based on the calculated imaging layer (S310).

2. The method as set forth in claim 1, wherein the first generating step is performed using a pressure transducer (202).

3. The method as set forth in claim 1, further comprising the step of performing the scan using a motion platform (206), (S312), wherein the motion platform (206) preferably includes an x-ray source (206-2) and an x-ray detector (206-3) and wherein further preferably the x-ray detector (206-3) is a solid-state sensor.

4. The method as set forth in claim 3, further comprising the step of transmitting a signal obtained by the scan to an image processing system (208; 400) (S.314).

5. The method as set forth in one of the claims 1 to 4, further comprising the step of generating tooth location information using the generated signal (S306).

6. The method as set forth in one of the claims 1 to 5, wherein the anatomical region of interest represents at least one of a dental arch and a location of a maxilla and a mandible.

7. A panoramic x-ray system (200), comprising:
a pressure transducer (202) adapted to generate a signal representing a pressure map of an anatomical region of interest;
a processor (204; 400) adapted to calculate an imaging layer based on the generated signal; and
a motion platform (206) adapted to perform a scan using the calculated imaging layer,
wherein the processor (204; 400) programs the motion platform (206) based on the calculated imaging layer.

8. The system (200) as set forth in claim 7, wherein the motion platform (206) includes an x-ray source (206-2) and an x-ray detector (206-3), wherein preferably the x-ray detector (206-3) is a solid-state sensor.

9. The system (200) as set forth in claim 7 or 8, wherein the anatomical region of interest represents at least one of a dental arch and a location of a maxilla and a mandible.

10. A method of conducting a panoramic x-ray examination, comprising the steps of:
generating a signal representing a pressure map of an anatomical region of interest; and
matching an imaging layer to the anatomical region of interest using the generated signal.

11. A method of conducting a panoramic x-ray examination, comprising the steps of:
generating an optical impression image of an anatomical region of interest; calculating an imaging layer based on the optical impression image; and
generating a scan based on the calculated imaging layer.

12. The method as set forth in claim 11, wherein the first generating step is performed using an intra-oral camera.

13. The method as set forth in claim 11 or 12, further comprising the step of performing the scan using a motion platform (206), (S312).

14. A method of conducting a panoramic x-ray examination, comprising the steps of:
generating a signal representing an anatomical region of interest; and
matching an imaging layer to the anatomical region of interest using the generated signal.

15. The method as set forth in claim 14, wherein the generating step is performed using an x-ray image.

16. The method as set forth in claim 14, wherein the generating step includes using a plurality of position-sensitive sensors creating the signal representing the anatomical region of interest.

17. The method as set forth in claim 14, wherein the generating step includes using a pressure transducer (202) creating the signal representing the anatomical region of interest.
